# EUROPEAN PATENT APPLICATION

(11) **EP 4 700 381 A2**
(43) Date of publication of application: **25.02.2026**
(21) Application number: 25227501.1
(22) Date of filing: 29.12.2023
(51) Int. Cl.: G01N 27/00

(54) **ELECTRODE FOR THE RECORDING AND MONITORING OF PHYSICAL, CHEMICAL OR BIOLOGICAL SIGNALS**

(62) Divisional of application: 23220777.9
(71) Applicant: Instituto Tecnológico de la Energía, 46980 Paterna Valencia (ES)
(72) Inventor: GARCIA CARMONA, Laura, VALENCIA (ES); BUAKI SOGO, Mireia, VALENCIA (ES); VERGAS GARCÍA, Marta, VALENCIA (ES); GARCÍA PELLICER, Marta, VALENCIA (ES); QUIJANO LÓPEZ, Alfredo, VALENCIA (ES)
(74) Representative: Lozano Alonso, Jose

(57) **Abstract**

The present invention relates to an electrode for the recording and monitoring of physical, chemical and/or biological signals, configured for use as a biosensor in medical and non-medical applications. The electrode comprises a flexible polymer membrane forming a support that includes at least one synthetic or natural biocompatible component combined with at least one carbon-based material, providing electrical conductivity, flexibility and mechanical stability. The flexible configuration allows adaptation to body surfaces and tissues, enabling wearable and in vivo applications with adequate biocompatibility. The electrode further comprises at least one biological and/or chemical recognition element, allowing its use as a sensor for chemical, electrical and/or biological parameters. The recognition element may be selected from proteins, enzymes, peptides, antibodies, nucleic acid sequences, CRISPR systems, ionophores or molecularly imprinted polymers. The biocompatible component is chitosan and the carbon-based material is carbon black.

## Description

### TECHNICAL FIELD

The present invention is part of the technical sector of electrodes for the monitoring of physical, electrophysiological, electrochemical, chemical or biological signals, which allow monitoring of different parameters, both in medical and/or non-medical environments.

In particular, the present invention describes a flexible polymeric electrode, which allows to support biological material for the realization of electrophysiological, physical and/or chemical measures, while ensuring electrical conductivity and sufficient biocompatibility to guarantee its safety in prolonged use, which, in addition, does not require the application of gels when used superficially, in direct contact with the user's skin.

### BACKGROUND OF THE INVENTION

Electrodes are used for the monitoring of parameters such as the recording of electrical pulses in living organisms. The most common applications are the electrodes used in pacemakers, electrocardiograms or electrostimulators.

In the latter applications, it is necessary to install in vivo electrodes, where the interaction between the electrodes and their host determines their viability in the body, especially in the long term.

There are many materials that are supposed to be biocompatible and potentially suitable to be implanted in living tissues, however, living tissues and organisms often respond by causing epithelialization, making their use difficult.

In this sense, one of the strategies to avoid this type of phenomenon would be the design of flat and flexible electrodes, without edges, and with rounded shapes, that have a small thickness.

Currently, polymeric materials are widely known, which offer numerous advantages for the design of electrodes, both for their lightness, malleability, corrosion resistance and low cost, as well as for their electrical, mechanical and optical properties.

Particularly, when they are used in electrochemical applications, their behavior is that of a semiconductor, so it is necessary to increase the conductivity of polymers. One of the most used strategies is the preparation of composites, that is, materials composed of a heterogeneous mixture of materials; including nanomaterials that improve the properties of polymers.

On the other hand, for the preparation and development of bioelectrodes, different types of conductive organic polymers are used, but their use in the development of certain implantable or wearable devices is limited by their biocompatibility, low conductivity or affinity for biomolecules, and reduced possibility of adapting to different configurations.

That is why the use of organic materials in the development of bioelectrodes has been considerably extended, particularly the use of synthetic materials, or renewable materials obtained from natural resources, commonly referred to as biopolymers.

The use of synthetic polymers as support materials has the advantages of allowing the selection of the monomers that compose it, depending on the final application of the electrode itself, including or not biological material as incorporated enzymes. The type of monomers is determined by their structure and properties, such as solubility, porosity, stability, as well as their resistant and mechanical properties. In addition, they allow the modification of chemical groups for the subsequent functionalization of the electrode.

However, the use of polymers as support elements for the biological material, presents the limitation that the synthesis involves a slow, laborious process that involves a high cost.

On the other hand, biopolymers, that is, polymers of natural origin, have a plurality of properties due to their own nature, such as biodegradability, low toxicity and an excellent affinity for proteins, which makes them optimal materials for the immobilization of enzymes and other elements of biological recognition, minimizing the negative effects in terms of the stability of the enzyme. In addition, its biocompatibility properties allow its use in applications for implantable or wearable devices.

An advantage offered by biopolymers is that they are obtained through simple procedures, being sometimes obtained from by-products of industrial processes, which reduces costs and frames them in the field of the circular economy.

Biopolymers include proteins such as albumin, and materials of biological origin such as collagen, cellulose, keratin, carrageenan, chitin, chitosan or alginate.

Chitosan is the most widely used biopolymer as an enzyme immobilization support, due to its excellent biocompatibility properties and its low cost, in addition to its ability to be configured in a multitude of morphologies.

Within bioelectrodes, there are numerous manufacturing techniques that include chitosan, the most used being the compact tablets of carbonic materials, rigid carbon electrodes or metals such as gold or platinum, on which additional layers of different materials are deposited, in order to modify their properties and maximize the electronic transfer or load of immobilized biological material.

However, these manufacturing modes are unsuitable to be integrated into the human body, since most of them are rigid, with low conductivity or are not biocompatible since they are mainly used in the development of biosensors for in vitro analysis.

That is why it is necessary to develop flexible systems that can be used in in vivo applications, that have sufficient conductivity, can adapt to tissues and body surfaces, without causing allergic or toxic reactions in the individual, as well as being able to withstand the deformations and tensions of the daily routine.

Therefore, the development of flexible and biocompatible bioelectrodes, which allow the immobilization of biological recognition elements, and that have conductive and electronic transfer capacity to build applicable biosensor electrodes, is of particular interest.

In this sense, the most developed strategies are based on the use of biopolymers and carbonaceous materials to increase the conductive capacity. Among the most prominent strategies are the Buckypaper, the use of electrospun materials, and polymer membranes.

The Buckypaper is based on the use of macroscopic sheets formed from carbon nanomaterials that, despite not being composed of polymeric materials, their configuration is flexible and have charge transfer capacity and biocompatibility. The biggest advantage they have is that it is not necessary to include an additional support that acts as a current collector. In addition, they are lightweight materials and can be processed with different shapes and sizes. However, they have low conductivity, which is insufficient in certain applications.

Electro-spin materials are based on the use of large-length nanofibers, with constant diameter and heterogeneous composition, with high porosity and large surface area, which are also biocompatible and with high mechanical strength. Its advantage is that, due to the heterogeneity of materials, properties adapted to the enzyme and its particular application can be obtained. However, they present a very laborious synthesis process.

Polymeric membranes have a large surface area, which allows the efficient bonding of immobilized biological material. In addition, they have good porosity, comprising the pores of a well-defined size and structure, which facilitate the immobilization of biomolecules, not only on the surface of the support itself, but also inside the pores. Likewise, they have a good diffusion capacity and mechanical stability.

Its flexibility characteristics make it easy to prepare with different morphologies, adapting to any application.

The need to apply these elements in in viva systems makes it necessary to make advances in the design that improve the density of power obtained, the output voltage, the operational stability of biodevices and the appropriate characteristics for the immobilization of biological recognition elements. In addition, it is necessary that they have other properties such as high contact area, electrical conductivity and mechanical stability.

In this sense, we can find in the state of the art different documents that refer to this type of electrodes.

We can find, for example, the document WO2018218969, which describes the development of an electrode composed of a substrate of mainly non-woven fibers, which uses a layer of adhered conductive gel for use in an electrocardiogram and electroencephalogram apparatus.

In particular, the polymeric material is soluble in water, which may be chitosan among others, which includes carbon black as a filling material, obtaining a gel that guarantees excellent biocompatibility and sensitivity.

We can also find document US 20210268152, which describes a conductive and biocompatible membrane capable of transporting the electrical potential of a cardiac impulse. This membrane comprises gelatin, a conductive polymer and a biocompatible component, where the conductive polymer can be polypyrrole, and the biocompatible component can be chitosan, among others.

We can also find document WO 2020107345, which describes an implantable flexible electrode, which comprises at least one functional layer that can be of conductive materials, such as graphene, carbon nanotubes or polypyrrole. This electrode requires the use of polymer hydrogel, such as chitosan, in the electrode stimulation area.

We can also find document US 20010031988, which describes a biological electrode for bioelectric control and/or bioelectric stimulation, which consists of an electrode of biodegradable material, such as chitosan, which is used as a matrix that acquires conductivity after adding a conductive filler, which can be carbon black. Likewise, it is indicated that the electrode requires a conductive gel.

We can also find different scientific publications that refer to the use of flexible electrodes such as the one entitled "Flexible and Conductive Bioelectrodes Based on Chitosan-Carbon Black Membranes: Towards the Development of Wearable Bioelectrodes" by Mireia Buaki-Sogó, Laura Garcia-Carmona, Mayte Gil-Agustí, Marta García-Pellicer and Alfredo Quijano-López published in 2021, which describes the synthesis of a chitosan membrane with carbon black.

### SUMMARY OF THE INVENTION

The flexible polymeric electrode with conductive capabilities that the invention proposes is configured, therefore, as a remarkable novelty within its field of application, since, according to its implementation and exhaustively, the objectives indicated are achieved, being the characteristic details that make it possible and that distinguish them, conveniently included in the final claims that accompany this description.

The present invention tries to solve the problems indicated above, in particular, it tries to offer a flexible, conductive polymeric electrode that allows to support biological material for the realization of electrophysiological, physical and/or chemical measures, ensuring enough biocompatibility to guarantee its safety in prolonged use, which in addition, does not require the application of gels when used superficially, in direct contact with the user's skin.

Specifically, the present invention describes a flexible polymeric membrane with conductive capacities, which comprises a support made as a flexible polymeric membrane, with at least a biocompatible support material, to which a carbon-based material is added, preferably a nanomaterial, to improve the conductive properties.

In addition, the flexible polymer membrane object of the present invention can be doped with a conductive polymer and/or a metal element that increases the conductivity of the electrode.

Based on the previous description, the invention proposes a particular application of the polymeric electrode with conductive capabilities.

The invention describes an electrode for the registration and monitoring of physical, chemical or biological signals, which will include a biological and/or chemical recognition element, allowing its use as a sensor of chemical, electrical and/or biological parameters.

This electrode comprises a support made as a flexible polymer membrane, with at least one biocompatible support material, to which a carbon-based material is added, preferably a nanomaterial, to improve its conductive properties, in addition, it could be doped with a conductive polymer.

In particular, the chemical and/or biological recognition element is chosen from among those in the following list: a protein and/or an enzyme and/or a peptide and/or an antibody and/or a nanoantibody and/or a DNA sequence and/or an RNA sequence and/or a CRISPR system and/or any biological recognition element derived from the previous elements or an ionophore or a molecular printing polymer (MIPs) or synthetic molecular recognition polymers or any chemical recognition element derived from the above elements.

In particular, any of the electrodes described above may include components with elastic properties, which allow their elastic deformation without breakage or erroneous operation.

Likewise, they can also include elastic fixing media, plastics and/or any other type of flexible support. In this way, apart from the common uses of electrodes in medical fields, they can also be used as wearable or portable devices, being able to overcome those disadvantages of the state of the art, such as the wearing of watches in certain sports, or the impossibility of using chest bands.

In this way, a flexible polymeric electrode is achieved with improved characteristics of biocompatibility in dermal and oral models, flexibility, adaptability, and conduction of electricity, which is obtained from a simple procedure free of toxic and low cost products, which also includes a composition that can be framed within the scope of the circular economy in terms of waste recovery, since chitosan can be obtained from the skins of seafood and crustaceans; and compostability, both of chitosan and carbonated material.

The flexible polymer electrode with conductive capabilities, and the set of elements described, represent an innovation of structural and constitutive characteristics unknown until now, reasons that, together with its practical usefulness, provide it with sufficient foundation to obtain the privilege of exclusivity that is requested.

### PREFERRED EMBODIMENTS OF THE INVENTION

In the following detailed description of the preferred embodiments, reference is made to the attached drawings that are part of this report, and in which specific preferred embodiments in which the invention can be carried out are shown as an illustration.

### Flexible polymer electrode with conductive capabilities

In a first mode of realization, the present invention describes a flexible polymer electrode with conductive capabilities, which is configured from a flexible polymer membrane, with at least one biocompatible support material, which includes:
- A synthetic or natural biocompatible component
- A carbon-based material, which is preferably a nanomaterial.

In a preferred embodiment of the previous one, the polymeric electrode is doped with a conductive polymer, such as polypyrrole or PEDOT, and/or a metallic element, such as gold or silver metallic nanoparticles, for example; that improve the conductivity of the electrode.

In a preferred embodiment, it is described that the support material is chitosan, which is used in an amount between 1% and 2% by weight, in a solution of acetic acid between 0.02 mol/l and 0.7 mol/l.

In the same way, in another preferred embodiment, the porous material based on coal is carbon black, which is used in an amount between 10% and 150% by weight, with respect to the used mass of chitosan.

In particular, the amount of carbon black used will be exactly 100% by weight with respect to the mass of chitosan used.

By using the above compounds, within the ranges of values given, the applicants have managed to increase the conductive capacity of the electrode by between 10 and 100 orders of magnitude, depending on the amount of carbon black used.

In a preferred embodiment, the invention will include a component with elastic properties, such as an elastomer or similar, so that the electrode can be used in applications that include elastic elements.

Because the inclusion of the elements described, the invention achieves a biocompatible electrode, with better electronic transfer, and good mechanical properties of traction, torsion and bending. In addition, it is a simple synthesis free of toxic products that can be produced at a low cost.

### Electrode for recording and monitoring physical, chemical or biological signals

In a second way of carrying out the invention, an electrode for the recording and monitoring of physical, chemical or biological signals is described, which includes a flexible polymer membrane with a biocompatible support material that comprises at least one synthetic or natural biocompatible component and at least one coal-based material, with at least one biological and/or chemical recognition element, configured to be used as a sensor of chemical, electrical and/or biological parameters.

In a preferred embodiment, the biocompatible component is chitosan, and the carbon-based material is carbon black.

In addition, preferably, the combination of the biocompatible component and the carbon-based material is doped with a conductive polymeric material, such as polypyrrole or PEDOT; and/or with a metal element, such as the use of gold or silver nanoparticles.

Preferably, the combination of chitosan and carbon black will be doped with a conductive polymeric material, particularly polypyrrole, which improves electronic transfer with respect to conventional electrodes.

In particular, the chemical and/or biological recognition element is chosen from the following list: a protein and/or an enzyme and/or a peptide and/or an antibody and/or a nanoantibody and/or a DNA sequence and/or an RNA sequence and/or a CRISPR system and/or any biological recognition element derived directly or indirectly from the above elements.

Likewise, the biological recognition element can be chosen from among those in the following list: an ionophore or a molecular printing polymer (MIPs) or synthetic molecular recognition polymers or any chemical recognition element derived directly or indirectly from the above elements.

According to the previous realizations of sensors, both electrophysiological and physical, chemical or biological, a sensor is obtained that does not require the use of conductive gel when measuring the parameters.

Likewise, it is a sensor with superior oral and dermal biocompatibility compared to conventional commercial electrodes.

Sufficiently described the nature of the present invention, as well as the way to put it into practice, it is not considered necessary to make its explanation more extensive so that any expert in the field understands its scope and the advantages that are derived from it, stating that, within its essentiality, it may be put into practice in other forms of realization that differ in detail from the one indicated by way of example, and to which the protection that is obtained will also reach, provided that its fundamental principle is not altered, modified or changed.

## Claims

1. Electrode for the recording and monitoring of physical, chemical or biological signals **characterized in that** includes a flexible polymer membrane with a biocompatible support material that comprises at least a combination of a synthetic or natural biocompatible component and a carbon-based material, with at least one biological and/or chemical recognition element that is configured to be used as a biosensor of chemical, electrical and/or biological parameters.

2. Electrode for the registration and monitoring of physical, chemical or biological signals according to the previous claim **characterized in that** the biocompatible component is chitosan and the carbon-based material is carbon black.

3. Electrode for the registration and monitoring of physical, chemical or biological signals according to any of the preceding claims **characterized in that** the combination of the synthetic or natural biocompatible component and the carbon-based material is doped with a conductive polymer and/or a metallic element.

4. Electrode for the registration and monitoring of physical, chemical or biological signals according to the previous claim **characterized in that** the chemical and/or biological recognition element is a protein and/or an enzyme and/or a peptide and/or an antibody and/or a nanoantibody and/or a DNA sequence and/or an RNA sequence and/or a CRISPR system and/or any biological recognition element derived directly or indirectly from the above elements.

5. Electrode for the registration and monitoring of physical, chemical or biological signals according to any of the preceding claims **characterized in that** the biological recognition element is an ionophore or a molecular printing polymer (MIPs) or synthetic molecular recognition polymers or any chemical recognition element derived directly or indirectly from the above elements.
